# EUROPEAN PATENT APPLICATION

(11) **EP 4 837 413 A1**
(43) Date of publication of application: **11.11.2026**
(21) Application number: 26387056.0
(22) Date of filing: 28.04.2026
(51) Int. Cl.: A01M 1/20, A01M 17/00, A23B 2/00, A23B 7/005, A61L 2/04, G06N 7/00, G16C 20/30, G16C 20/70

(54) **METHOD FOR AUTOMATICALLY CALCULATING THE DISINFESTATION TIME FOR DRIED FRUITS**

(30) Priority: 05.05.2025 GR 20250100330
(71) Applicant: University of Thessaly, 38221 Volos (GR)
(72) Inventor: VLONTZOS, Georgios, 38446 Volos (GR); ATHANASIOU, Christos, 38333 Volos (GR); VASILEIOU, Marios, 38333 Volos (GR); SAKKA, Maria, 38222 Volos (GR); GKOURGKOUTA, Marina, 50100 Kozani (GR); KYRGIAKOS, Leonidas-Sotirios, 38222 Volos (GR); KLEISIARI, Christina, 41222 Larisa (GR)
(74) Representative: Panagiotidou, Effimia

(57) **Abstract**

The present invention is a method for automatically calculating the total minimum required duration of disinfestation for products with low moisture content, such as dried fruits, legumes, tobacco, spices, and grains, by determining the duration of heat penetration and the duration of thermal treatment with minute-level precision, based on the characteristics of the specific products and the target insects in the specific geographic region where it is applied. The method is installed at the site where the disinfestation will take place and provides thermal disinfestation of products, combining advanced modeling techniques with a user support system and the use of sensors (301). The invention achieves the integration of penetration modeling into thermal treatment, enabling precise predictions of the duration of the disinfestation, accurate to the minute, required for the product pallets to reach the target temperature (temperature at the shortest disinfestation duration) during disinfestation, using iterative methods.

## Description

The invention relates to the field of post-harvest disinfestation of low-moisture foods using various technologies.

Low-moisture foods, such as dried fruits, legumes, tobacco, spices, and grains, are widely consumed due to their high nutritional value, long shelf life, and ease of storage and transport. However, during storage and distribution, insect infestation poses a significant challenge, making disinfestation essential to ensure product quality and safety. During disinfestation, pesticides and fumigants are used to control insects, potentially leading to residues and raising significant concerns regarding food safety and environmental sustainability. To address the side effects of pesticides, current practices primarily rely on non-chemical disinfestation methods. Non-chemical disinfestation methods in low-moisture foods include, among others, methods that use radiation, modified atmosphere (MA), and dielectric heating. In this context, thermal treatments have emerged as a more sustainable solution for disinfestation, as they are more effective processes with less negative impact on the physicochemical properties of low-moisture foods undergoing treatment, on human health and the environment. The effectiveness and efficiency of thermal treatment depend on various factors, including the type of insects, the physicochemical properties of the food, and the uniformity of heat distribution within the food.

The technical problem with thermal disinfestation (whether cold or hot) of low-moisture foods using current state of the art is that it does not accurately calculate the processing time required for the complete elimination of insects at specific temperatures, as it relies solely on empirical estimates or generalized protocols that do not take into account the actual conditions of each application. This leads either to under-treatment, with the risk of insect survival, or to over-treatment, which increases energy costs and may adversely affect product quality and alter their nutritional characteristics.

The uneven distribution of heat within packages/pallets of low-moisture food products during processing, variations in the physicochemical properties of the products, and the differing resistance of insects to heat across geographical regions make it difficult to accurately calculate the time required for disinfestation.

Furthermore, the influence of environmental parameters, such as the initial temperature of the pallet and the physicochemical properties of the products, such as their moisture content and thermal conductivity, make it necessary to accurately calculate the time required to achieve complete insect elimination at all points on the pallet.

Therefore, a key technical challenge of the current state of the art is the accurate calculation of the minimum required disinfestation duration based on a temperature range, taking into account the physicochemical properties of the specific products and the specific insects found in the specific geographical region.

In the current state of the art, the most common thermal disinfestation techniques include thermal treatment with hot or cold air, thermal treatment in a controlled atmosphere, dielectric and microwave heating, and heating with infrared radiation.

In current state of the art documents, a protocol for such cold disinfestation treatment should provide quarantine safety against a specific insect with minimal impact on the quality of the commodity, as noted in the publication [Follett, P.A., Neven, L.G. Current trends in quarantine entomology. Annu. Rev. Entomol. 2006, 51, 359-385; Yahia, E.M. Effects of insect quarantine treatments on the quality of horticultural crops. Stewart Postharvest Rev. 2006, 2, 1-18]. A comprehensive disinfestation process, first involving exposure to 1.5 °C for 3 days and then exposure to a controlled atmosphere at 25 °C, could eliminate insects without negatively affecting the quality of "Clemenules" mandarins [Palou, L., Jacas, J.A., Marcilla, A., Alonso, M., del Rio, M.A. Physico-chemical and sensory quality of 'Clemenules' mandarins and survival of the Mediterranean fruit fly as affected by complementary cold and carbon dioxide quarantine treatments. Postharvest Biol. Technol. 2008, 48, 443-450]. The study by Hofman *et al.* reported that a comprehensive treatment with hot water at 41 °C for 25-30 minutes, or at 42 °C for 25 minutes following 16 days at 1 °C, could improve the external and internal quality of avocado fruit and control fruit flies [Hofman, P.J., Stubbings, B.A., Adkins, M.F., Meiburg, G.F., Woolf, A.B. Hot water treatments improve 'Hass' avocado fruit quality after cold disinfestation. Postharvest Biol. Technol. 2002, 24, 183-192]. Other data showed that 6 °C for 3 days, followed by 1 °C for 16 days, improves the quality of ripe "Hass" avocado fruit without negative effects, while also providing disinfestation of B. *tryoni* [Hofman, P.J., Stubbings, B.A., FAdkins, M., Corcoran, R.J., White, A., Woolf, A.B. Low temperature conditioning before cold disinfestation improves 'Hass' avocado fruit quality. Postharvest Biol. Technol. 2003, 28, 123-133]. Among the treatments accepted for quarantine measures, cold disinfestation is an effective approach for controlling pests found in fresh fruit [U.S. Department of Agriculture, Animal and Plant Health Inspection Service. Treatment Manual]. However, the treatment must meet the requirements for minimal impact on the quality of the product, as excessive exposure of the product to extreme temperatures (high or low) degrades the product, unlike the present invention, which calculates the exact disinfestation time using a combination of modeling and empirical data.

The same technical problem is addressed in U.S. Patent No. US6171561B1, "Mechanically Loaded Direct Air Circulation Commodity Disinfestation Chamber"; however, it uses a heating chamber without temperature sensors to dynamically calculate the total disinfestation time, in contrast to the present invention, which uses temperature sensors and advanced modeling techniques to accurately calculate the disinfestation time in both a heat chamber and a cold chamber (refrigerator).

The disadvantages of the current state of the art are, in particular, as follows:
- Some methods of the current state of the art for calculating the required time are based on static empirical models or generalized protocols, which do not adjust to the specific processing conditions. For example, the disinfestation time is calculated without taking into account critical variables such as the thermal conductivity of the dried fruit, the thickness of the pallet, or the actual temperature inside the product layers. This leads either to excessive thermal treatment, which can negatively affect the quality of the dried fruit, or to insufficient treatment, which leaves room for insects to survive, especially when the treatment is not tailored to specific target insects. In particular, excessive exposure to high or low temperatures, which often results from inaccurate and empirical estimates, can cause nutrient loss, texture deterioration, and changes in the taste or color of dried fruits.
- Some systems incorporate temperature sensors to monitor thermal distribution; however, they lack dynamic algorithmic models enabling adaptive calculation of processing time based on operating conditions.
- Most existing applications do not incorporate automated mechanisms for dynamically adjusting thermal penetration and thermal treatment based on product properties, such as size, moisture content, and thermal conductivity, and rely on general estimates or empirical data. The absence of such mechanisms increases the risk of errors and reduces process efficiency.
- Some systems do not adequately account for the specific properties of each type of dried fruit or the insects that may affect it. The lack of specialization limits the accuracy and effectiveness of disinfestation, while the uniformity of the result is not guaranteed.
- Due to the aforementioned drawbacks, these systems typically consume significant amounts of energy to achieve the desired results. This increases the process's environmental footprint as well as operational costs for producers.
- Existing systems of the current state of the art lack adequate real-time monitoring of temperature and thermal penetration in the product being processed. This shortcoming limits the ability to adjust the process to actual conditions.

The present invention provides a method for automatically calculating the total minimum required duration for the disinfestation of products, determining the duration of thermal penetration and the duration of thermal treatment with minute-level precision, based on the characteristics of the specific products and the target insects in the specific geographic region where it is applied.

The method provides thermal disinfestation of products by combining advanced modeling techniques with a user support system and the use of sensors. The invention integrates penetration modeling into the thermal treatment process, enabling precise predictions of the duration of disinfestation, accurate to the minute, required for product pallets to reach the target temperature (temperature at the shortest disinfestation duration) during disinfestation, using iterative methods. The method is initially installed at the disinfestation site by the installer, and then handed over to the user for operation. The key parameters used throughout the application of the method, from installation to use, are the type of product, the initial temperature of the pallet (of the product), the final disinfestation temperature, the type of target insects, the volume of the product, and a thermal degradation coefficient (proportionality constant), which depends on the physicochemical properties of the product and on empirical measurements in relation to the product's volume.

The method employs iterative calculations, followed by parameterization of the key variables by the system installer. The method automatically adjusts the disinfestation process to specific operational requirements, in the shortest possible time, with minute-level precision, taking into account the different dimensions of the pallets and suggesting to the user the temperature range within which disinfestation can be achieved. Additionally, the method is implemented using a temperature monitoring system in the storage facility for low-moisture food products.

Application of the method during the postharvest stage provides the advantage of improved precision (minute-level accuracy), effectiveness (100% mortality), and adaptability (to the requirements of each specific warehouse, using existing equipment and targeting specific insect species) in the disinfestation of dried fruit. The integration of combined modeling, which utilizes thermal treatment and thermal penetration models, makes the method applicable to all types of products with low moisture content and to all target insects in the specific geographical area and facilities in question. These models, which predict the duration of thermal treatment, ensure that every part of the product pallets reaches the required temperature for disinfestation. The disinfestation method and the system (technical equipment) that implements it, allow for monitoring, data analysis, and real-time decision-making, thus offering the user an immediate solution to any problem that may arise during disinfestation. Furthermore, the method allows users to input various parameters, run simulations based on combined models, and obtain useful information and recommendations for optimizing the disinfestation process. Calculating the appropriate temperature and the duration of the pallet's exposure to it with minute-level precision, tailored to the specific storage conditions, ensures the complete elimination of all insects during disinfestation without the use of any chemicals. More specifically, the monitoring and recording of the critical parameters of the disinfestation process is a central feature of the method, contributing to the proper management of resources-namely, energy savings, increased productivity and availability, as well as the precise adjustment of thermal treatment to prevailing conditions, thereby minimizing the risk of human error. This capability offers the advantage of saving time and energy compared to the current technique, which often results in excessive or insufficient heating or excessive or insufficient exposure time for the products. At the same time, data logging facilitates the analysis of disinfestation results and provides useful information for the continuous improvement of the process. The present method has the added advantage of being combinable with other methods, such as the use of controlled atmospheres or physical barriers, thereby reducing the amount of chemical agents required by these methods. Thus, users ensure maximum effectiveness in disinfestation, while minimizing environmental impacts and dependence on chemicals.
Figure 1: Overview of the system's operational steps.
Figure 2: Modeling of the system's thermal treatment penetration.
Figure 3: Overview of the sensor system.
Figure 4: Schematic of the pallet showing the placement of temperature sensors.
Figure 5: Steps for system operation by the user.
Figure 6: Graphical representation of the thermal penetration function of temperature (°C) over time (hours).
Figure 7: Table 1 - Specifications table for the disinfestation method.
Figure 8: Table 2 - Experimental data from a cold chamber sensor in a test storage facility.
Figure 9: Table 3: Parameters used to calculate the heat transfer coefficient.
Figure 10: Table 4: Mortality rates of *Oryzaephilus surinamensis* at distinct temperatures and exposure times.

The invention is defined by the Claims.

The invention comprises a combination of technical equipment, the incorporation of empirical data and parametric simulation techniques for the accurate calculation of the duration of thermal penetration of a specific temperature into the pallet, as well as the duration of thermal treatment, which are necessary for the complete disinfestation of dried fruit pallets. The thermal penetration time is the duration required for the entire pallet to reach a specific temperature, while the thermal treatment time is the duration required for the complete disinfestation of the pallet at that temperature. Thermal treatment is carried out by exposing product pallets to specific temperatures for a predetermined duration to eliminate specific insects. Factors such as exposure temperature, exposure duration, and the life stage of the insects (adults, nymphs, eggs, larvae) are taken into account. Different insects have different tolerance levels to heat and cold, which must be taken into account when designing thermal treatment protocols. In the example of the invention described here, dried fruits are used; however, the invention can be applied in the same way to other stored agricultural products and foods with low moisture content, such as legumes, tobacco, spices, grains, etc.

The pallet is used as an illustrative example of the method's scope of application, as the method is applied in exactly the same way even when the fruits are not on pallets. Wherever reference is made in the present text to an application example involving a pallet, it concerns pallet dimensions of approximately 1.50 m in height × 1.20 m in width × 1.20 m in length, but of course the method applies in the same way to pallets of other dimensions used in current practice. Furthermore, the method is applied in the same way if the products to be treated are not placed on pallets but in other packaging, more suitable for the nature of each product, such as, for example, bags, boxes, or bulk products stored in containers, depending on the storage and transport requirements of each type of low-moisture food.

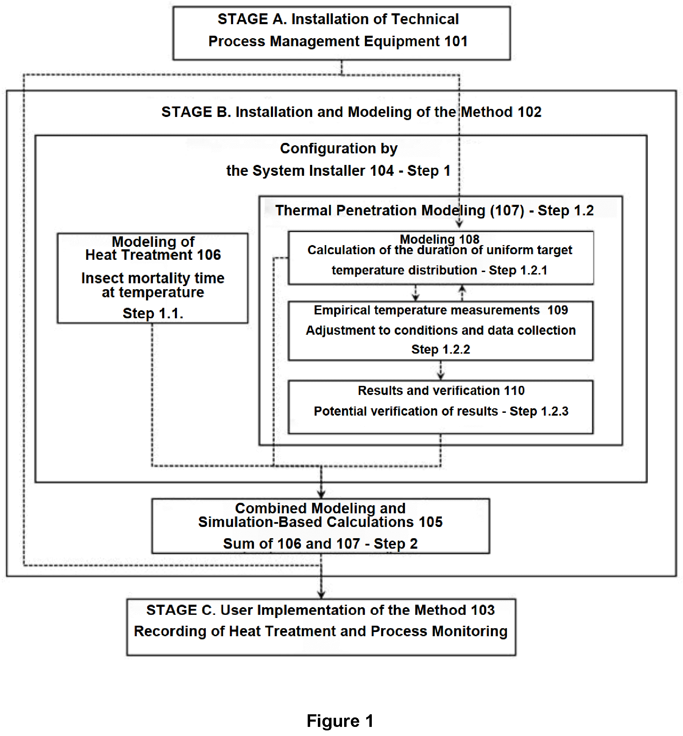

In summary, the method is implemented and carried out in the following stages (Fig. 1):
STAGE A. Installation of technical process management equipment by the system installer (101).
STAGE B. Installation and modeling of the method (102) with the following steps:
   STEP 1.: Configuration by the installer (104) with the following sub-steps:
      STEP 1.1.: Modeling of thermal processing (106)
      STEP 1.2.: Modeling of thermal penetration (107)
      STEP 1.2.1.: Modeling (108)
      STEP 1.2.2.: Empirical temperature measurements (109) with Data Collection, Calculation of the thermal decomposition coefficient, and Model Calibration
      STEP 1.2.3.: Results and verification (110)
   STEP 2.: Combined Modeling and Simulation-Based Calculations (105).
STAGE C. User Application of the Method (103).

### Technical Specifications for Application of the Method

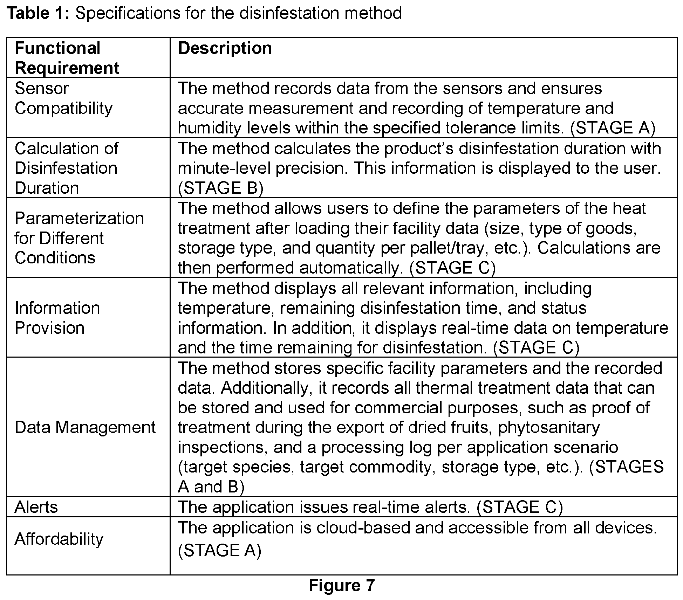

The table of specifications for the disinfestation method (Figure 7-Table 1) lists the specific tasks and functions that the application is designed to perform in the dried fruit warehouse environment.

### Specifically:

STAGE A. Installation of process management technical equipment (101) by the system installer

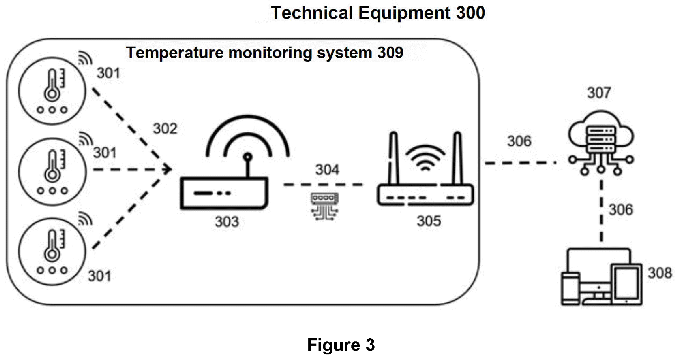

Figure 3 illustrates the technical equipment required for the application of the method in an overview of its architecture and operational framework. The technical equipment is used/installed in the dried fruit warehouse where the disinfestation method will be applied.

The technical equipment (300) includes a temperature monitoring system (309), a central server (307), and a user interface (308) (e.g., a computer) for monitoring and configuring the process. The temperature monitoring system (309) consists of at least two temperature sensors (301), which are capable of measuring the chamber temperatures, as well as a local server (303) with software, a router (305), and a communication interface (304) connecting them. Communication (302) between temperature sensors (301) and the local server (303) is carried out via a communication protocol, potentially using Zigbee. The router (305) connects the server (303) to the central server (307) where the application is stored. Communication between the router (305), server (307), and user interface (308) is carried out via the internet (306). Communication between the local server (303) and the router (305) is carried out via communication protocols (304), potentially using Wi-Fi or Ethernet.

Furthermore, the temperature monitoring system (309) includes at least two temperature sensors (301) capable of wireless communication with the server (303), which sensors are installed on at least one pallet (at least 2 sensors per pallet) in the area where disinfestation will take place and which are connected to the server (303) that collects the temperature data, processes it in real time, and transmits it via communication protocols (304) and the network (306).

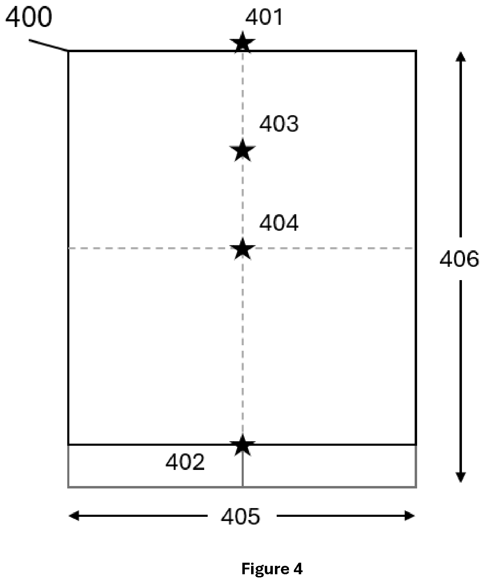

Specifically, as shown in Figure 4, which depicts a pallet, for the method to be properly implemented, the sensors (301) are positioned with at least one sensor on the outside of the pallet (point 401 or 402) and at least one sensor in the center of the pallet (point 404). The method achieves maximum performance when four sensors are placed, namely two sensors on the outside of the pallet at diametrically opposite points (points 401 and 402) and one more sensor at the midpoint (point 403) of the distance separating the outer sensor from the sensor placed at the center of the pallet (point 404):

| | |
|---|---|
| • Sensor 1 (point 401): | Top, outer surface of the pallet [0/4 distance (406)] |
| • Sensor 2 (point 402): | Bottom, outer surface of the pallet [4/4 distance (406)] |
| • Sensor 3 (point 403): | Top, inside near the center of the pallet [1/4 distance (406)] |
| • Sensor 4 (point 404): | Center of the pallet [1/2 distance (406)]. |

### STAGE B. Installation and method modeling (102)

### STEP 1.: Configuration by the system installer (104)

Figure 1 illustrates the steps by which the thermal treatment penetration modeling method is performed.

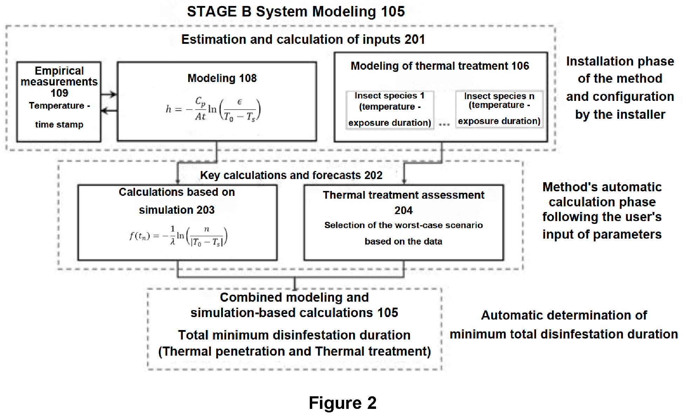

As shown in Figures 1 and 2, the method applies modeling techniques (102) to calculate the minimum total duration of thermal disinfestation of dried fruits with minute-level precision. To achieve this, the following must be determined and calculated:
1. The duration and temperature of the thermal treatment (disinfestation), i.e., how much time is needed to kill the target insects and at what temperatures (Step 1.1).
2. The duration of thermal penetration, i.e., how long it takes for the entire pallet to reach the chamber temperature (Step 1.2).

In summary, the thermal treatment is first modeled based on current technical (106), and data regarding the duration of pallet exposure for disinfestation at specific temperatures for specific target insects, as entered by the installer, are stored in the system, as described below in Step 1.1. The following is the thermal penetration modeling (107), Step 1.2., derived from its modeling (108) (Step 1.2.1.), based on empirical temperature measurements (109) (Step 1.2.2.) which are used in the above modeling (108) (Step 1.2.1.), and include actual temperature measurements as a function of time from sensors (301). These measurements are used to calculate the thermal degradation coefficient *λ*, which is used in the thermal penetration model described in Step 1.2.1, which calculates the duration of thermal penetration. The system installer may then verify the accuracy of the thermal penetration model in Step 1.2.3. Finally, using the thermal treatment and thermal penetration data, the system calculates the minimum total disinfestation time, accurate to the minute, as described in Step 2. Specifically:

### STEP 1.1.: Modeling of thermal treatment based on current technique (106)

In this step, the specific target insects are first identified, usually based on the location where the method will be applied. Next, the "mortality data" for these specific insects are entered into the system; that is, for each target insect species, the following is recorded: a) the temperature range-whether very high or very low for the insects' survival-at which the specific insects are killed, and this data is stored; and b) the required exposure duration for each of the specific temperatures within the range to achieve complete disinfestation. These mortality data for the specific insects within this temperature range whether they exist as data from the current standard or are collected from controlled thermal treatment experiments, are used in the application of the method when adjusting the model parameters (105) by a specialized entomologist through on-site sampling and subsequent laboratory analysis. To utilize this data, it is stored in the central server database (307) of the system, and the temperature is correlated with a specific frequency-for example, 0.5 degrees Celsius-with the required exposure time of the pallet for disinfestation, per temperature (e.g., the following information will be stored as data: disinfestation at 0 °C will require 20,160 minutes, disinfestation at -0.5 °C will require 19,020 minutes, disinfestation at -1 °C will require 18,120 minutes, and so on). More specifically, this data is stored in a table format with the main columns comprising: (a) insect species, (b) temperature (in °C), and (c) exposure duration for 100% mortality (in minutes). In conclusion, this step records the total range of temperatures at which disinfestation is feasible (defined as the "disinfestation temperature range").

### STEP 1.2.: Modeling of thermal penetration (107)

In this step, differential equations are formulated to capture the cooling or heating process of the pallets when exposed to different environmental conditions. Next, empirical correlations and sensor data are incorporated to refine the method's models. Finally, the final model is determined, which calculates the time required for the pallet's temperature to reach the chamber temperature, from its outer surface to its center. Specifically:

### STEP 1.2.1.: Modeling ((108) and (203))

Determining the time required for the pallet's temperature to reach the chamber's temperature (refrigerator or heating chamber) from the temperature of the warehouse in which it was located involves examining many factors, including the initial temperature of the pallet (warehouse temperature) and the chamber temperature. Accordingly, the above description can be expressed as the Differential Equation (DE) Eq. (1): $\frac{{dT}_{p} \left(t\right)}{dt} = - λ \left({T}_{p}\left(t\right)-{T}_{s}\right)$ (1) where *Tₚ*(*t*) is the pallet temperature at time t, and $λ = \frac{hA}{{C}_{p}} > 0$ is a thermal degradation coefficient, which depends on empirical measurements (109).

Eq. (1) defines the process by which the pallet undergoes cooling/heating through the gradual removal of heat to an unlimited cooling/heating source (refrigerator or heating chamber). Solving the differential equation in Eq. (1) yields the following Eq. (2), assuming that the pallet's temperature at a given time *t =* 0 is *Tₚ*(0) = *T*₀ equal to the pallet's initial temperature, which is typically the warehouse temperature: $T \left(t\right) = {T}_{s} + \left({T}_{0}-{T}_{s}\right) {e}^{- λt}$ (2)

The equation demonstrates an exponential relationship between the pallet's temperature and the cooling time, accounting for the thermal properties of the materials, including thermal conductivity and specific heat capacity.

Given the above, it is difficult to determine the exact duration required for the temperature *Tₚ*(*t*) to reach *Tₛ* since, theoretically, *Tₚ*(*t*) approaches *Tₛ*, asymptotically. Therefore, a very small threshold *ε* ≤ |*Tₛ* - *T*₀| was set in order to define a practical endpoint. As an example, this limit was set to be a temperature difference of 0.1°C. To find the time required for the pallet to reach the chamber temperature, Eq. is defined *T(t) = Tₛ + ε* and solved (2) for : *t:* ${T}_{s} + ϵ = {T}_{s} + \left({T}_{0}-{T}_{s}\right) {e}^{- λt}$ (3)

Subtracting *Tₛ* from both sides: $ϵ = \left({T}_{0}-{T}_{s}\right) {e}^{- λt}$ (4)

Dividing both sides *T*₀ - *Tₛ* and taking the natural logarithm of both sides, we have: $ln \left(\frac{ϵ}{{T}_{0} - {T}_{s}}\right) = - λt$ (5)

Finally, the solution for t: $t = - \frac{1}{λ} ln \left(\frac{ϵ}{{T}_{0} - {T}_{s}}\right)$ (6)

In short, t represents the time required for the pallet to reach within *ε* of the ambient temperature *Tₛ*. This equation is based on various parameters:
- Initial temperature (*T*₀): The initial temperature of the pallet before it is placed in the cold or thermal chamber.
- Chamber temperature (*Tₛ*): The temperature to which the chamber is set, selected from the range of disinfestation temperatures.
- Thermal degradation coefficient (*λ*): Determined by the heat transfer coefficient h, the pallet surface area *A,* and the specific heat capacity *Cₚ* of the pallet as $λ = \frac{hA}{{C}_{p}}$.
- Threshold temperature difference (*ε*): A small value indicating how close the pallet temperature must be to the chamber temperature for the process to be considered complete.

The thermal penetration modeling algorithm is designed to iteratively calculate the time required for the entire pallet to reach various temperatures within the "temperature range for disinfestation" (Step 1.1.) at a specific frequency, the same as that of Step 1.1, for example 0.5 °C. Specifically, the duration of thermal penetration is calculated at each intermediate temperature between the initial temperature of the pallet and the chamber temperature (defined as "intermediate temperatures"). This iterative calculation of time allows the determination of the disinfestation duration for specific temperatures, as specific intermediate temperature points are automatically identified to minimize the disinfestation duration. For example, if the initial temperature of the pallet (storage temperature) is 20 °C and it is placed in a refrigerator set to 10 °C (chamber temperature), the algorithm will evaluate the thermal treatment results for increasing temperatures close to the target. This involves calculating the time required for the pallet to reach each of the intermediate temperatures, such as 10 °C, 10.5 °C, 11 °C, 11.5 °C, 12 °C, 12.5 °C, etc. Consequently, the algorithm will calculate all the intermediate temperatures and temporarily store them in a list.

The reason for this iterative approach is to correlate the intermediate temperatures of this step with the temperatures from Step 1.1, since the next step will determine the total duration required to achieve insect elimination for each intermediate temperature, which is calculated as the sum of the thermal treatment duration and thermal penetration.

Using Eq. (6) and the above data, it can be concluded that the threshold *ε* can be used as a repeat temperature step.

Consequently, for thermal penetration, the model is formulated as follows: $f \left({t}_{n}\right) = - \frac{1}{λ} ln \left(\frac{n}{\left|{T}_{0}-{T}_{s}\right|}\right)$ (7) where *n* = 0.1, 0.6, 1.1, 1.6, ...., |*Tₛ* - *T₀*|

### STEP 1.2.2.: Empirical temperature measurements (109)

In this step, empirical temperature measurements are performed, taking into account the actual conditions prevailing in the space, using temperature sensors (301) that were installed on the pallets during the installation of the technical equipment (STAGE A), in order to feed the thermal penetration model (108), (Step 1.2.1). These measurements provide temperature data, which are used to incorporate empirical data and improve the accuracy of the calculations. Specifically, the following steps are performed:
1.2.2.1. Data collection: The initial temperature of the pallet is measured: First, the installer selects any temperature from the data stored in the central server database (307) during Step 1.1, within the "disinfestation temperature range", based on the capabilities of the disinfestation facility's equipment -whether a cold chamber or a heat chamber- and the characteristics of the products, at which disinfestation can be achieved. This temperature is provisionally defined, for the purpose of test measurements, as the "chamber temperature". Next, at least one test pallet, on which the sensors (301) have been mounted, is placed inside the chamber so that empirical measurements can be taken by recording the temperature inside the chamber. The sensors (301) have been installed as described above in step "A. Installation of Process Management Equipment (101)". The pallet remains in the disinfestation chamber for as long as necessary to reach the temperature defined as the "chamber temperature". Throughout the pallet's stay in the chamber, and until the pallet reaches the "chamber temperature". continuous measurements are taken, at least one measurement per minute: a) the pallet's temperature, based on sensor readings (301), until it reaches the chamber temperature; and b) the time (time stamp) of the temperature reading, and the data is stored and integrated into the system. After data recording is complete, the test pallet, which carries the sensors, is removed from the chamber.
1.2.2.2. Calculation of the thermal degradation coefficient: Using the collected data and knowing the positions of the sensors within the pallet, the thermal degradation coefficient *λ* is calculated using Eq. (6). $t = - \frac{1}{λ} ln \left(\frac{ϵ}{{T}_{0} - {T}_{s}}\right)$ (8)
   To accurately model the thermal penetration of the pallets during thermal treatment, the heat transfer coefficient h must be calculated, which is then incorporated into the calculation of the thermal degradation coefficient *λ*.
   Using (a) Eq. (6), (b) the empirical data, and (c) analyzing the thermal degradation coefficient in $λ = \frac{hA}{{C}_{p}}$*,* the heat transfer coefficient *h* is calculated as follows: $t = - \frac{{C}_{p}}{hA} ln \left(\frac{ϵ}{{T}_{0} - {T}_{s}}\right)$ (9)
   Rearrangement for solution for *h*: $h = - \frac{{C}_{p}}{At} ln \left(\frac{ϵ}{{T}_{0} - {T}_{s}}\right)$ (10)
   Where *Cₚ* is the specific heat capacity of the pallet and A is the surface area of the pallet (m²).
1.2.2.3. Model Adaptation: The theoretical model is fitted with the empirically derived λ, ensuring that it accurately reflects the thermal behavior observed in the experimental data.

The data collected in this step are used to calculate the thermal degradation coefficient, which is incorporated into the thermal penetration model (108) during the execution of Step 1.2.1. to improve its accuracy.

The installer also places temperature sensors (301) inside the chamber and/or in the surrounding area of the chamber, which are connected to the technical equipment as described above in the technical equipment installation section.

### STEP 1.2.3.: Results and verification (110) by the installer

After completing the above steps, the results are compared with actual temperature measurements to validate the accuracy of the models. For example, the installer may compare the temperature indicated by the result of Step 1.2.1 with the actual pallet temperature provided by the sensors (301). If the calculations from Step 1.2.1 do not match the measurements, the method is repeated starting from Step 1.2. The application is monitored in real time via the temperature monitoring system (309) (Figure 2), ensuring that processing conditions remain within the specified limits (Step 1.1.) for the effective elimination of insects. The control procedure specified in this step is performed based on the parameters defined in Step 1.2.1.

### STEP 2.: Combined Modeling and Simulation-Based Calculations (105).

In this step, the correlation between thermal penetration and thermal treatment is established to achieve complete disinfestation of the pallet (Figure 2). More specifically, using the thermal penetration data ((107) and (203)) from Step 1.2.1. and the thermal treatment data (106) from Step 1.1, an automatic calculation of the temperature at the shortest disinfestation duration is performed; that is, the "target temperature" is determined based on the shortest possible disinfestation duration.

More specifically, to calculate the minimum total disinfestation duration, the following parameters and variables must be taken into account:
1. Initial pallet temperature *T*₀ (°C): The initial temperature of the pallet before treatment, prior to its entry into the chamber, a value provided either by the user or by the installed sensors (301).
2. Chamber temperature *Tₛ* (°C): The temperature of the processing environment (e.g., refrigerator or thermal chamber), a value provided either by the user or by the installed sensors (301).
3. Heat degradation coefficient *λ*: A coefficient that determines the rate of temperature change, which was calculated during system setup in Step 1.2.1.
4. Target insect characteristics: Thermal tolerance and mortality rates for the specific target insects and their life stages, calculated in Step 1.1.

Given the above parameters, the total time required for the disinfestation process is first calculated for each temperature (T) within the "disinfestation temperature range" and the "intermediate temperatures", denoted as *Duration(T),* which is the sum of the thermal penetration duration from Step 1.2.1 and the thermal treatment duration from Step 1.1: *Duration(T)* = *Thermal Penetration Time(T) + Thermal Treatment Time (T).* This calculation generates a preliminary list consisting of temperatures and the disinfestation duration for each temperature. From this list, the temperature with the shortest duration is selected, which is defined as the minimum total time required for the disinfestation process; that is, it is defined as the target temperature (T).

For example, suppose a pallet is initially at 20 °C, the chamber temperature is 0 °C, and the disinfestation temperature range is -10 °C to 3 °C. Instead of considering only the final temperature of 0 °C, the algorithm evaluates "intermediate temperatures" such as 0 °C, 0.5 °C, 1 °C, 1.5 °C, 2 °C, 2.5 °C, and 3 °C, as described in Step 1.2.1. That is, it calculates the total disinfestation duration for each of the above intermediate temperatures (thermal treatment duration + thermal penetration duration) and saves a temporary list consisting of temperatures and the disinfestation duration for each temperature. This helps determine the minimum disinfestation duration, as there may be cases where achieving a slightly higher or lower temperature than the chamber temperature can result in a shorter total disinfestation time. In the event that disinfestation is performed for more than one target insect simultaneously -which have been defined by the method installer in Step 1.1 and selected by the user- the thermal treatment data (204) are automatically determined by the system, by selecting the overlapping range of disinfestation temperatures and the longest thermal treatment duration among the target pests.

In summary, upon completion of Step 2, by combining the heat penetration duration from Step 1.2.1, as shown in Eq. (7), with the heat treatment duration from Step 1.1, as analyzed in this step, the following is obtained: 1) the total minimum duration, accurate to the minute, required for the proper application of the method, from the moment the pallet is placed in the chamber until the completion of the disinfestation process; and 2) the "target temperature", which may differ from the chamber temperature.

### STAGE C. Application of the method by the user (103)

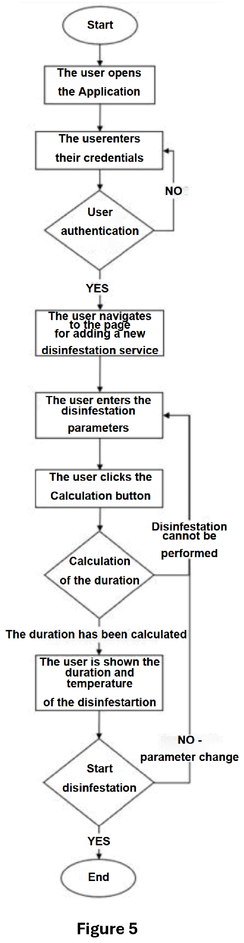

Once the installation and modeling of the method's system (102) are complete, as per Stage B, the user performs the actions described below for a set of pallets (Figure 5).

Step C.1.1.: The user opens the application (on), enters their credentials, authenticates, and logs into the system.

Step C.1.2.: Navigates to the page for adding a new disinfestation.

Step C.1.3.: Fills in the disinfestation parameters, specifically:
C.1.3.1. the Pallet ID number, for one or more pallets;
C.1.3.2. the type of fruit to be treated;
C.1.3.3. the size and dimensions of the pallet(s) based on the dimensions specified by the end user;
C.1.3.4. the target insect species from those entered into the system in Step 1.1; specifically, the user has the option to select all insects provided by the system, or only some of them depending on the specific season in which the method is applied;
C.1.3.5. the initial temperature of the pallet, which is measured outside the chamber before it is introduced into the chamber, and the number of the sensor providing this measurement;
C.1.3.6. the chamber temperature, i.e., the temperature to which the pallet will be exposed throughout the entire disinfestation process, and the number of the sensor providing this measurement.

Step C.1.4.: The user selects the "Calculate" button to perform the disinfestation calculation, which indicates to the user the duration and temperature required for disinfestation. Specifically, when the "Calculate" button is clicked, the system calculates and displays to the user: 1) the "total minimum disinfestation duration", i.e., the minimum total time required for the disinfestation process; and 2) the "target temperature", as described in Step 2.

If the user enters a temperature at which, based on the data stored in Step 1.1, disinfestation is not feasible, the system displays an error message and suggests an acceptable temperature range within which disinfestation can be achieved (disinfestation temperature range).

If the user does not wish to start the disinfestation process and wishes to change the parameters, they have the option to do so in this step, in which case they restart the process from Step C.1.4.

### Step C.1.5.: The user starts the disinfestation by selecting the "Start Disinfestation" button

The system records the disinfestation (all the above data) and notifies the user via email upon completion. At the same time, the user has access to all disinfestation data at any time, as they can monitor the process in real time, since the application can provide live updates on temperature, humidity, treatment duration, and other relevant measurements. Furthermore, the inclusion of a countdown timer for each disinfestation process further enhances the user's ability to monitor progress and make informed decisions, such as terminating or adjusting the process if necessary. The ability to delete or archive records ensures that the system remains organized and that historical data is easily accessible for review. In addition to real-time monitoring, the application offers robust capabilities for managing historical disinfestation data. This feature is essential for users who need to review and analyze past disinfestation jobs. The page provides detailed records of completed procedures, including timestamps, parameters, and results. This archived data can be vital for compliance with regulatory requirements, audits, and improving future disinfestation strategies based on past performance.

### Use Example - Case Study

Field experiments and case study: To validate the simulation findings, a series of experiments was conducted in warehouses belonging to the Union of Agricultural Cooperatives. This example was implemented using a Raspberry Pi 4, a Sonoff Zigbee ZBdongle-E Smart Hub, and temperature-humidity sensors, specifically the Sonoff SNZB-02 and SNZB-02P models.

The Raspberry Pi 4 serves as the central server for managing data acquisition and storage. Wireless communication between the sensors and the Raspberry Pi is facilitated by the Sonoff Zigbee ZBdongle-E Smart Hub, using the Zigbee protocol. Key to the system's functionality are the Sonoff SNZB-02 and SNZB-02P temperature-humidity sensors, installed inside the warehouse. These sensors, equipped with Zigbee capabilities, continuously monitor environmental conditions, transmitting real-time data to the Raspberry Pi for processing and analysis. Finally, the use of Home Assistant software leverages the system's capabilities, offering a comprehensive set of functions for data logging, storage, and visualization.

### Utilization of thermal treatment data

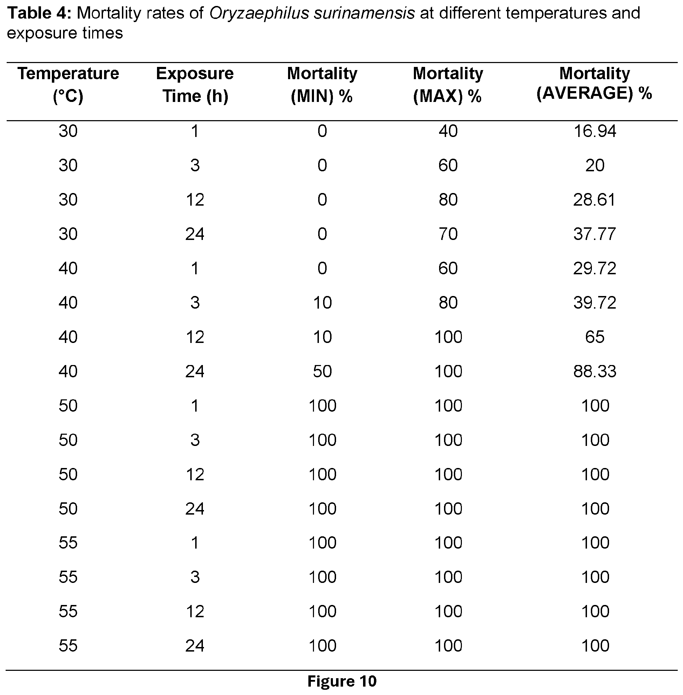

In this case study, mortality data from the saw-toothed grain beetle, *Oryzaephilus surinamensis* (L.) (Coleoptera: Silvanidae) were used. The entomological experiments conducted to calculate mortality, whose data were used, are explained in detail in the article Sakka, M. K., Gourgouta, M., & Athanassiou, C. G. (2024). Efficacy of Extreme Temperatures on All Life Stages of the Mediterranean Flour Moth, Ephestia kuehniella Zeller (Lepidoptera: Pyralidae). *Agronomy,* 14(6), 1307). Table 4/Figure 10 presents the total mortality measurements (combined: data for adults, pupae, eggs, and larvae) for heat treatment as an example.

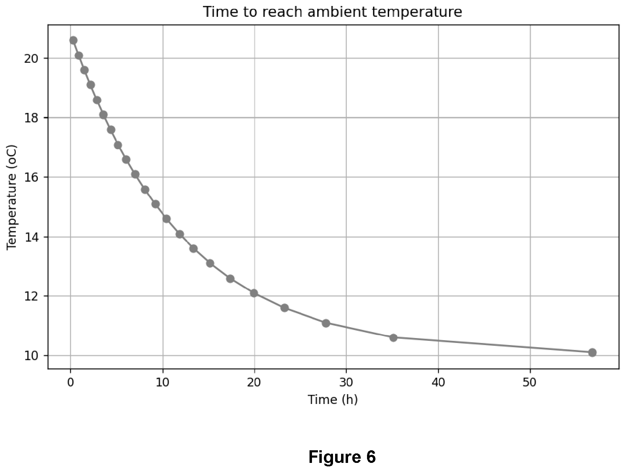

These data are further illustrated in Figure 6. This diagram shows the relationship between temperature, exposure time, and mortality. In order to calculate intermediate values as a continuous set rather than discrete values, the linear interpolation method is used for the average values.

### Data collection

Accurate data collection is important for validating and calibrating the heat penetration model. Data is collected through measurements from temperature sensors.

To fully monitor temperature changes across the pallet during heat treatment, the sensors were placed at the recommended locations as described above during the installation of the Process Management Equipment (101) by the system installer, and are shown in Figure 4.

Placing the sensors in this manner ensures that a representative range of temperatures is recorded from the outer surfaces to the inner core of the pallet, providing a complete understanding of how heat penetrates the pallet.

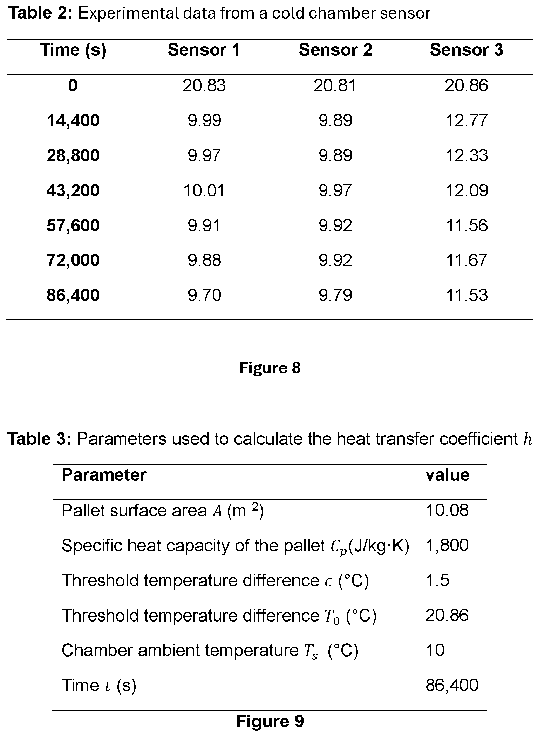

During the heat treatment process, the sensors record temperature data at regular intervals. Table 2 (Figure 8) summarizes the recorded temperatures (in °C) at key time points for each sensor in an experiment, as an example.

These empirical data show the evolution of temperature over time and help improve the heat transfer coefficients and the overall accuracy of the model, or, the temperature-versus-time data, which will be used for thermal penetration.

### Calculation of the heat dissipation coefficient

The following parameters based on actual conditions *h* were used to calculate the heat transfer coefficient (Table 3/Figure 9).

Using $λ = \frac{hA}{{C}_{p}}$ Eq. (6), and the parameters in Table 3/Figure 9, the heat transfer coefficient *h* is calculated as: *h* = 0.00409148 W/*m*²K.

Using this *h* value to calculate $λ = \frac{hA}{{C}_{p}}$*, λ* = 2,29123 · 10⁻⁵, which will be used to update the thermal penetration model using Eq. (6), as follows: $t = - 43644.7133 ⋅ ln \left(\frac{ϵ}{\left|{T}_{0}-{T}_{s}\right|}\right)$ (11)

### Thermal Penetration Application - Example

Following Step 1.2. and using Eq. (7), as an example, the Python programming language is used to visualize the results of the thermal penetration function using the parameters in Table 3/Figure 9. The graph generated by Eq. (7) illustrates the relationship between the iteration steps n, which represent the temperature deviation from the chamber temperature, and the time *f*(*tₙ*) required for the pallet to reach a specific temperature difference from the chamber temperature (Figure 6). As n increases from 0.1 to the absolute temperature difference |*Tₛ* - *T*₀|*,* the function *f*(*tₙ*) decreases logarithmically. This indicates that initially, when the temperature difference is significant, the rate of temperature change is rapid, resulting in a higher *f*(*tₙ*)*.* However, as the temperature difference decreases, the rate of change slows down and the value *f*(*tₙ*) decreases, approaching zero. The graph in Figure 6 illustrates the heat treatment process, which determines the exact time required for the pallet to reach various specific temperatures.

The method addresses the specific needs of disinfestation processes in storage environments for products with low moisture content, such as dried fruits, legumes, tobacco, spices, and grains. The application's functions are organized into several key areas, each of which is critical for the successful management and implementation of disinfestation processes.

The invention is applicable during the pre-packaging disinfestation stage in companies, organizations, or cooperatives that process dried fruits, legumes, tobacco, spices, grains, and, in general, foods with low moisture content.

### Definitions of terms used in this Description:

- Thermal treatment: is carried out by exposing pallets of dried fruit to specific temperatures for a predetermined duration to eliminate specific insects.
- Thermal penetration: refers to the process by which the entire pallet reaches a certain temperature from another, that is, how heat is transferred from the outer part to the center.
- Thermal treatment time: is the duration required for the complete disinfestation of the pallet at a specific temperature.
- Thermal penetration time: is the duration required for the entire pallet to reach a specific temperature.
- Disinfestation temperature: range is the total range of temperatures at which disinfestation is feasible.
- Chamber temperature: is the temperature to which the chamber is set, selected from the range of disinfestation temperatures.
- Target temperature: is the temperature at which total insect elimination is achieved in the shortest time.
- Intermediate temperatures: is the duration of thermal penetration at each intermediate temperature between the initial temperature of the pallet and the chamber temperature.
- Empirical data: is the information on temperature as a function of time, to be used for thermal penetration.
- Total minimum disinfestation: duration is the minimum total time required for the disinfestation process (including the duration of heat treatment and thermal penetration).

## Claims

1. A method for the disinfestation of low-moisture foodstuffs, performed using at least two temperature sensors, in real time, **characterized by** minimizing the total duration of thermal disinfestation of the food at specific temperatures with minute precision and comprising the following stages:
STAGE A. Installation of technical equipment for process management by the system installer (101), wherein at least one sensor is placed on the outside of the pallet (point 401 or 402) and at least one sensor in the center of the pallet (point 404),
STAGE B. Installation and modeling of the method (102) with the following steps:
STEP 1.: Configuration by the installer (104) with the following sub-steps:
STEP 1.1.: Modeling of thermal treatment (106) by entering mortality data, where the mortality data include:
(a) insect species;
(b) temperature (in °C);
(c) exposure time of food packages/pallets for 100% mortality in minutes at specific temperatures, demonstrating the range of disinfestation temperatures.
STEP 1.2.: Modeling of thermal penetration (107)
STEP 1.2.1.: Modeling (108) and (203) in which the duration of thermal penetration is calculated at each intermediate temperature between the initial pallet temperature and the chamber temperature, using as parameters the initial pallet temperature, the chamber temperature, and the thermal decay coefficient, and setting the threshold temperature difference
STEP 1.2.2.: Empirical temperature measurements (109)
by collecting data through successive measurements at predetermined time intervals using sensors (301) and storing the pallet temperature using the sensors (301) from the initial temperature until it reaches the chamber temperature, along with the time stamp of the temperature reading;
by calculating the thermal decay coefficient; and
by fitting the model, incorporating the coefficient into the model
STEP 1.2.3.: Exporting results and performing a check (110)
STEP 2.: Combined Modeling and simulation-based calculations (105) in which the following is provided: 1) the total minimum duration, accurate to the minute, required for the proper application of the method, from the moment the pallet is placed in the chamber until the completion of the disinfestation, and 2) the "target temperature" which may differ from the chamber temperature,
STAGE C. Application of the method by the user (103) using the following parameters:
- Pallet ID No., for one or more pallets,
- type of fruit to be treated,
- size and dimensions of the pallet(s),
- target insects from those entered into the system according to Step 1.1, selecting all insects included in the system (101), or only some of them depending on the specific season in which the method is applied,
- initial pallet temperature, when the pallet is outside the chamber, before it is introduced into the chamber, and the number of the sensor providing this measurement,
- chamber temperature and the number of the sensor providing this measurement,
calculation of the total minimum disinfestation duration, as described in Step 2, or, if a temperature at which disinfestation is not feasible is entered, the system displays an error message and suggests an acceptable temperature range within which disinfestation can be achieved, or, if the user wishes, performs a parameter change and restarts the process from the beginning of STAGE C,
the disinfestation process begins.

2. A method according to claim 1, **characterized in that** the maximum efficiency of the method is achieved when, in Step 1.2.2. of the empirical temperature measurements (109), four temperature sensors are placed, namely two temperature sensors on the outside of the pallet at diametrically opposite points (points 401 and 402) and one more temperature sensor at the midpoint of the distance (point 403) separating the outer sensor from the temperature sensor placed at the center of the pallet (point 404).

3. A method according to claims 1-2, **characterized in that** in STEP 2 Combined Modeling and Simulation-Based Calculations (105), initially, the total time required for the disinfestation process is calculated for each temperature within the disinfestation temperature range and the intermediate temperatures, where the total time is the sum of the thermal penetration duration, from Step 1. 2.1., and the duration of thermal treatment, Step 1.1,
a temporary list is created consisting of temperatures and the duration of disinfestation for each temperature, from which the temperature with the shortest duration, defined as the minimum total time required for the disinfestation process, is selected as the target temperature.

4. A method according to claims 1-3, **characterized in that,** for its application to more than one target insect simultaneously, which have been defined by the method installer in Step 1.1 and selected by the user, the method automatically determines the thermal treatment data (204) by selecting the overlapping range of disinfestation temperatures and the longest thermal treatment duration among the target insects.

5. A method according to claims 1-4, **characterized in that** the method upon completion of STAGE C, records the disinfestation data and notifies the user via email of its completion.

6. A method according to claims 1-5, **characterized in that,** the method upon completion of STAGE C, allows the user to access all disinfestation data at any time, with real-time monitoring of the process, real-time updates regarding temperature, humidity, processing duration, and other relevant measurements, and the ability to manage historical disinfestation data.

7. A system for automatically calculating the disinfestation time for low-moisture-content food, which fulfills claims 1-6 and which is **characterized in that** it comprises technical equipment (300) that includes at least
- a temperature monitoring system (309),
- a central server (307), and a user interface (308) for monitoring and configuring the process, wherein the temperature monitoring system (309) consists of at least two temperature sensors (301) connected to a local server (303) via software,
- a router (305), and
- a communication interface (304) between the local server (303) and the router (305), wherein the router (305) connects the server (303) to the central server (307) where the application is stored,
**and in that**
the sensors are installed on at least one pallet, with at least 2 sensors per pallet, in the area where disinfestation will take place, with at least one sensor positioned on the outside of the pallet (point 401 or 402) and at least one sensor in the center of the pallet (point 404), and wherein
the server is configured to process the data in real time and transmit it via communication (304) and network (306) protocols.
